# EUROPÄISCHE PATENTSCHRIFT

(11) **EP 3 242 539 B1**
(45) Veröffentlichungstag und Bekanntmachung des Hinweises auf die Patenterteilung: **13.10.2021**
(21) Anmeldenummer: 17168529.0
(22) Anmeldetag: 27.04.2017
(51) Int. Cl.: H05K 5/00, A61M 1/14, G06F 1/16, G06F 1/18, G02F 1/1333

(54) **VORRICHTUNG ZUR INTEGRATION EINES BILDSCHIRMS ODER MONITORS IN EIN GEHÄUSE**
APPARATUS FOR INTEGRATING A SCREEN OR MONITOR IN A HOUSING
DISPOSITIF D'INTÉGRATION D'UN ÉCRAN OU D'UN MONITEUR DANS UN BOÎTIER

(30) Priorität: 03.05.2016 DE 102016108201
(43) Veröffentlichungstag der Anmeldung: 08.11.2017
(73) Patentinhaber: B. Braun Avitum AG, 34212 Melsungen (DE)
(72) Erfinder: SCHÄFER, Oliver, 36286 Neuenstein (DE); SCHLEICHER, Christian, 36160 Dipperz (DE)
(74) Vertreter: Winter, Brandl - Partnerschaft mbB

(56) Entgegenhaltungen:
- WO-A1-2008/157200
- JP-A- H06 237 086
- US-A1- 2005 067 956
- US-A1- 2005 213 924
- US-B1- 6 437 238

## Beschreibung

Die Erfindung betrifft eine Vorrichtung zur Integration eines Bildschirms oder Monitors in ein Gehäuse und bezieht sich insbesondere auf eine Vorrichtung zur Monitorintegration über Kunststoffrahmen in ein Blechgehäuse, insbesondere in ein Blechgehäuse eines medizintechnischen Geräts oder einer Dialysemaschine.

Bei Maschinen zur extrakorporalen Blutbehandlung sind Monitorbaugruppen, welche entweder in statisch bestimmten tragenden Metallgehäusen integriert sind, die gleichzeitig eine sichtbare Gehäuseoberfläche darstellen, oder statisch bestimmte Metallrahmen, welche die Monitorkomponenten aufnehmen und als gehäusebildende Oberfläche eine Kunststoffblende aufweisen, bekannt.

Monitorgehäuse, welche beispielsweise aus einem Metallguss hergestellt werden, gewährleisten zwar geforderte mechanische Eigenschaften, Eigenschaften bezüglich EMV (elektromagnetischer Verträglichkeit) und funktionelle Integration, sind jedoch aufgrund hohen Fertigungsaufwands mit gegebenenfalls entsprechender Veredelung sehr teuer. Ein weiterer Nachteil dieser Lösungen besteht darin, dass bei einem tragenden Metallgehäuse das äußere gehäusebildende Design integriert ausgebildet ist und dadurch eine Mehrfachverwendung aufgrund verschiedener Designmerkmale und somit Unterschieden zu anderen Monitorbaugruppen nicht möglich ist.

Bei Systemen, die ein Kunststoffgehäuse verwenden, ist das mittels beispielsweise einem Spritzgussverfahren hergestellte Kunststoffgehäuse meist nicht alleine in der Lage, mechanische Eigenschaften, beispielsweise im Fall eines berührungsempfindlichen Bildschirms eine geforderte Ebenheit und/oder Steifigkeit gegenüber Verwindungen, sicherzustellen, welche jedoch notwendig sind, um eine störungsfreie Funktion zu gewährleisten. Daher bilden derartige Kunststoffgehäuse meist nur eine dekorative Hülle, während innenseitig ein metallischer Rahmen zur Aufnahme der Monitorbauteile zur Anwendung kommt. Ein weiterer Nachteil bei gehäusebildenden Bauteilen aus Kunststoff besteht in geringer elektromagnetischer Störfestigkeit. Ausreichende Störfestigkeit kann in diesem Fall nur über eine spezielle und zusätzliche Beschichtung auf dem Kunststoffteil erreicht werden.

In US 2005/0067956 A1 wird eine gattungsgemäße Baugruppe mit einem Monitor, einem Gehäuse und einem Tragelement zur Integration des Monitors in das Gehäuse offenbart, bei welcher das Tragelement durch Biegen hergestellt ist.

Gattungsfremde Monitorbaugruppen mit Tragelementen aus Kunststoff werden beispielsweise in US 2005/0213924 A1, JP H06-237086 A oder WO 2008/157200 A1 offenbart.

US 6,437,238 B1 offenbart ein PDA (Personal Digital Assistant), bei dem ein Bildschirm zwischen zwei jeweils mehrlagig ausgebildeten Gehäuseteilen eingeklemmt ist. Beide Gehäuseteile weisen jeweils eine Lage aus Metall und eine Lage aus Kunststoff auf.

Der Erfindung liegt daher als eine Aufgabe zugrunde, die vorstehenden Nachteile zu überwinden und eine verbesserte Vorrichtung zur Integration eines Bildschirms oder Monitors in ein Gehäuse bereitzustellen.

Speziell soll für insbesondere eine Dialysemaschine als ein Beispiel einer Maschine zur extrakorporalen Blutbehandlung eine solche Vorrichtung bereitgestellt werden.

Diese Aufgabe wird erfindungsgemäß gelöst durch eine Baugruppe mit einem Monitor, einem Gehäuse und einer Vorrichtung zur Integration des Monitors in das Gehäuse mit den Merkmalen des Anspruchs 1 und durch eine Dialysemaschine mit den Merkmalen des Anspruchs 14. Vorteilhafte Weiterbildungen der Erfindung sind Gegenstand der beigefügten Unteransprüche.

Der Erfindung liegt als eine allgemeine Idee zugrunde, alle Komponenten, welche für einen Informationstransfer und eine Interaktion relevant sind, wie ein berührungsempfindlicher Teil, ein Flachbildschirm (TFT), eine Statusanzeige, Bedienelemente (physisch ausgebildete Tasten und Schalter), Leiterplatten und dergleichen, in einem multifunktionalen Kunststoffrahmen aufzunehmen und in ein Blechgehäuse, welches separat bereitgestellt sein kann oder gleichzeitig ein Maschinengehäuse bilden kann, zu integrieren.

Materialeigenschaften und eine Geometrie des multifunktionalen Kunststoffrahmens gewährleisten gemeinsam mit den Materialeigenschaften des Blechgehäuses und weiterer Versteifungselemente, die in einer gestapelten Anordnung oder Sandwichbauweise angeordnet sind, eine ausreichende Steifigkeit für die Funktion der berührungsempfindlichen Komponenten (Touchpanel). Hierdurch erhöht sich die Funktionalität des Gehäuses. Neben an sichtbaren Teilen zu Tage tretenden dekorativen Elementen entfaltet das Gehäuse darüber hinaus eine tragende Wirkung für eine Gesamtfunktionalität der Touchpanel- und TFT-Integration. Die Sandwichbauweise mit einem Kunststoffrahmen ist kostengünstiger herzustellen als die bekannten Lösungen bei Maschinen zur extrakorporalen Blutbehandlung.

Die Integration in ein Metallgehäuse aus Blech eliminiert gegenüber Monitorgehäusen aus Kunststoff Einflüsse durch elektromagnetische Störungen und beseitigt dadurch die bei einem Kunststoffgehäuse notwendige zusätzliche EMV-Beschichtung. Außerdem besteht bei Verwendung standardisierter Schnittstellen die Möglichkeit der Integration in verschiedene Blechgehäuse, beispielsweise in ein separates Monitor-Blechgehäuse, welches z.B. drehbar auf ein Maschinengehäuse aufgesetzt ist, oder in ein korpusartiges Maschinengehäuse aus Blech. Somit können dieser Rahmen und die komplette Baugruppe als Gleichteil in verschiedensten Geräten verbaut werden. Gleichzeitig stellt der in beispielsweise einer Ausnehmung des Blechgehäuses stirnseitig teilweise sichtbare Kunststoffrahmen einen optisch abhebbaren Übergang zu dem lackierten Blechgehäuse dar.

Im Einzelnen werden die vorstehenden Vorteile realisiert und die Aufgabe gelöst durch eine Vorrichtung zur Integration eines Monitors in ein Gehäuse mit einem Tragelement, das aus einem Kunststoff hergestellt ist und eine Ausnehmung aufweist, die einer vorbestimmten sichtbaren Fläche des Monitors entspricht, wobei das Tragelement eine Vielzahl von Öffnungen, die in vorbestimmter Verteilung außerhalb der Ausnehmung zur Durchführung von Positionierungs- und/oder Halteelementen und Platzierung des Tragelements in vorbestimmter Position an einem Blechgehäuse, in welches das Tragelement aufnehmbar ist, angeordnet sind, und einen Rahmenabschnitt, der gegenüber einer Grundfläche des Tragelements auf einer Rückseite desselben vorsteht und zur Aufnahme von Komponenten des Monitors in Sandwichbauweise angeordnet ist, aufweist, wobei die in dem Rahmenabschnitt aufgenommenen Komponenten des Monitors an dem Tragelement festlegbar sind und das Tragelement an den Positionierungs- und/oder Halteelementen an dem Blechgehäuse festlegbar ist.

Erfindungsgemäß ist zumindest eine der Komponenten des Monitors mit einer definierten Eigensteifigkeit zum rückseitig flächigen Abstützen der im Rahmenabschnitt aufgenommenen Komponenten des Monitors ausgebildet und weist zumindest einen umfangsseitig angeordneten Festlegeabschnitt auf, der dazu angeordnet ist, die zumindest eine der Komponenten des Monitors an dem Tragelement festzulegen.

Erfindungsgemäß weist das Tragelement rückseitig vorstehend zumindest eine entlang eines Umfangs des Tragelements angeordnete erste Halteeinrichtung zur schraubbaren Festlegung zumindest eines Anbauteils, bspw. der Gehäuserückwand und/oder des Tragelements an dem Blechgehäuse auf, und/oder weist zumindest eine zweite, entlang eines Umfangs des Rahmenabschnitts angeordnete Halteeinrichtung zur schraubbaren Festlegung der zumindest einen Komponente des Monitors auf.

Bevorzugt ist die erste Halteeinrichtung ausgebildet, zumindest ein Anbauteil, bspw. eine Gehäuserückwand an dem Blechgehäuse schraubbar festzulegen.

Bevorzugt ist dabei die zumindest eine Komponente des Monitors dazu angeordnet, elektrische Konnektivität für zumindest eine elektronische Baugruppe mit Bezug zu den Komponenten des Monitors bereitzustellen und/oder die elektronische Baugruppe festzulegen.

Bevorzugt ist die Vorrichtung mit zumindest einem Versteifungselement versehen, das dazu angeordnet ist, das Tragelement mit den in dem Rahmenabschnitt aufgenommenen Komponenten des Monitors an dem Blechgehäuse festzulegen.

Bevorzugt ist das zumindest eine Versteifungselement dazu angeordnet, eine elektronische Baugruppe mit Bezug zu den Komponenten des Monitors festzulegen.

Bevorzugt besteht/bestehen das zumindest eine Versteifungselement aus einem Metall und kann schraubbar festgelegt werden.

Bevorzugt ist die zumindest eine Komponente des Monitors mit dem Tragelement in Kunststoff verschraubbar. Vorteilhaft kann hierbei die Komponente des Monitors ein bildgebendes Element, wie beispielsweise eine Flüssigkristall-Anzeigeeinrichtung oder TFT-Anzeigeeinrichtung, sein und über an dem bildgebenden Element angeformte Befestigungsstrukturen mit dem Tragelement in Kunststoff verschraubbar sein.

Bevorzugt ist das zumindest eine Versteifungselement mit zumindest einem Teil der Positionierungs- und/oder Halteelemente des Blechgehäuses, die aus Metall bestehen, verschraubbar.

Bevorzugt weist die Ausnehmung des Tragelements einen geringeren Umfang auf als eine Ausnehmung des Blechgehäuses, derart, dass stirnseitig des Blechgehäuses ein vorbestimmter Abschnitt des Tragelements die Ausnehmung des Blechgehäuses innenseitig rahmend sichtbar ist.

Bevorzugt beinhalten die Komponenten des Monitors zumindest ein berührungsempfindliches, transparentes und flächiges Element, das dazu angeordnet ist, eine Abdichtwirkung bereitzustellen und/oder Toleranzen auszugleichen, und ein bildgebendes Element, die gestapelt in dem Rahmenabschnitt des Tragelements angeordnet sind und einen berührungsempfindlichen Bildschirm ausbilden.

Bevorzugt sind das Tragelement, ein Dichtungselement, das zwischen dem Tragelement und einer der Komponenten des Monitors angeordnet ist, sowie die Komponenten des Monitors dazu angeordnet, vormontiert zu sein und als funktionsfähig anschlussbereite Baugruppe in das Blechgehäuse gesetzt und mit diesem verbunden zu werden.

Bevorzugt beinhaltet die Vorrichtung einen Subrahmen, der in den Rahmen des Tragelements einsetzbar und dazu angeordnet ist, die Größe des Rahmens und/oder der Ausnehmung des Tragelements zu verringern.

Insbesondere vorteilhaft kann eine Dialysemaschine dazu angeordnet sein, eine Vorrichtung zur Integration eines Monitors wie vorstehend beschrieben aufzuweisen, wobei das Blechgehäuse ein Gehäuse oder ein Korpus einer Dialysemaschine ist.

Die Erfindung wird nachstehend anhand eines bevorzugten Ausführungsbeispiels unter Bezugnahme auf die beigefügte Zeichnung näher beschrieben. Es zeigen:
Fig. 1 in einer vereinfachten Explosionsaufsicht eine Vorrichtung zur Integration eines Monitors in ein Blechgehäuse gemäß dem Ausführungsbeispiel; und

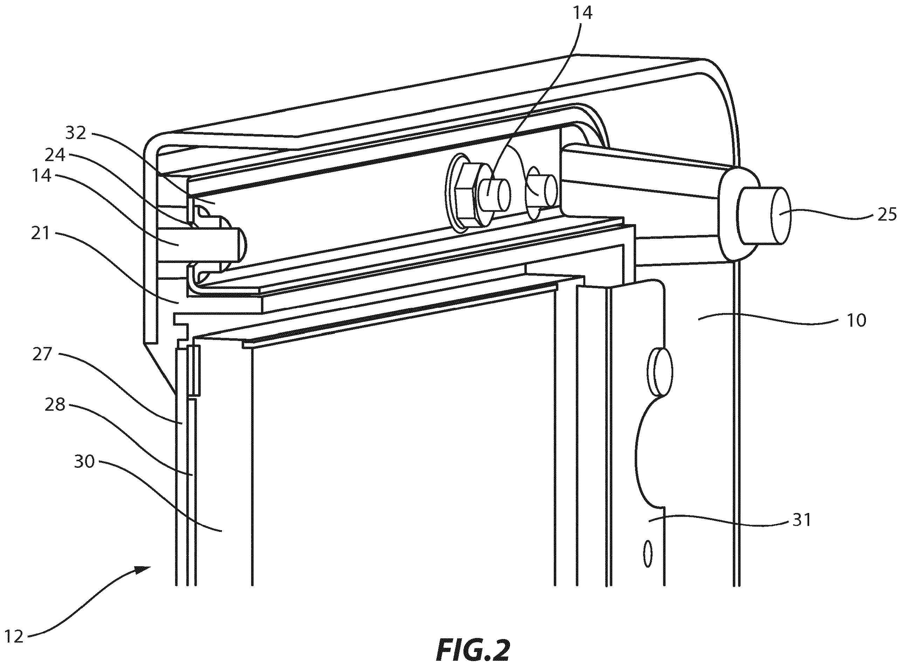

Fig. 2 eine schematische, ausschnittweise Ansicht der in Fig. 1 dargestellten Vorrichtung zur Integration eines Monitors in einem in das Blechgehäuse montierten Zustand.

In der nachfolgenden Figurenbeschreibung werden in den einzelnen Figuren gleiche oder gleichwirkende Elemente und/oder Komponenten gleich und/oder mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

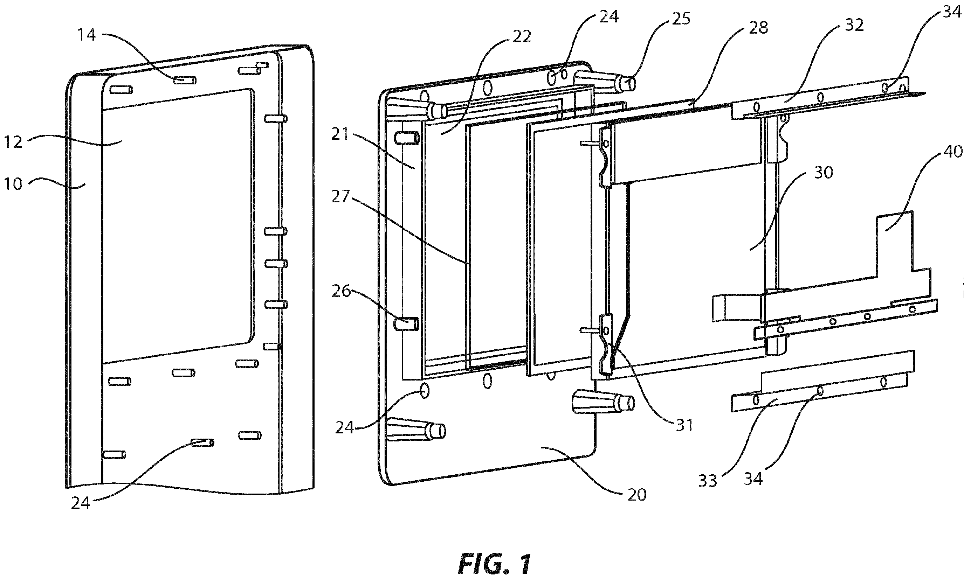

Wie in Fig. 1 gezeigt ist, weist ein Blechgehäuse 10, das beispielsweise ein Gehäuse oder ein Korpus eines medizintechnischen Geräts oder einer Dialysemaschine sein kann, eine Ausnehmung 12 auf, die eine Art Fenster bildet und dazu angeordnet ist, den Blick auf einen Bildschirm eines Monitors zu erlauben, der in das Gehäuse einbaubar oder an das Gehäuse anbaubar ist. Auf einer einem Geräteinneren zugewandten Rückseite des Blechgehäuses 10 sind vorzugsweise mehrere, beispielsweise stiftförmige, Erhebungen 14 verteilt angeordnet, die als Positionierungs- und/oder Halteelemente dienen und in Verbindung mit entsprechenden Öffnungen eines zu montierenden Anbauteils dessen Positionierung bezüglich der Gehäusewand in vorbestimmter Lage ermöglichen. Zur Konfiguration eines Halteelements weisen die stiftförmigen Erhebungen 14 bevorzugt eine Gewindeanordnung (Innengewinde (bei einer Hohlstiftform) und/oder Außengewinde) auf, an welcher das Anbauteil mittels beispielsweise einer Verschraubung festgelegt werden kann.

Ein Anbauteil der vorgenannten Art bildet gemäß dem Ausführungsbeispiel insbesondere ein Tragelement 20, das einen aus einem Kunststoff hergestellten Rahmen und als solcher eine Art Grundplatte mit einer Grundfläche bildet. Das Tragelement 20 weist ebenfalls eine Ausnehmung 22 auf, die zu der Ausnehmung 12 des Blechgehäuses korrespondiert und einer vorbestimmten sichtbaren Fläche des Monitors entspricht. Außerdem weist das Tragelement 20 außerhalb der Ausnehmung 22 eine Vielzahl von durchgehenden Öffnungen 24 auf, deren Lage und/oder Verteilung am Tragelement 20 jeweils den Positionen der Erhebungen 14 an dem Blechgehäuse 10 entspricht, so dass die Erhebungen 14 als Positionierungs- und/oder Halteelemente sowie zur Platzierung des Tragelements 20 in vorbestimmter Position an dem Blechgehäuse 10, in welches das Tragelement 20 aufnehmbar ist, durch die Öffnungen 24 führbar sind.

Die Ausnehmung 22 des Tragelements 20 umgebend bzw. umrahmend ist an dem Tragelement 20 ferner ein Rahmenabschnitt oder Rahmen 21 ausgebildet, der gegenüber der Grundfläche des Tragelements 20 auf dessen Rückseite vorsteht und zur Aufnahme eines Dichtungselementes 27 sowie von Komponenten 28 und 30 eines Monitors bzw. Bildschirms in Sandwichbauweise dient. Die Abmessungen (Höhe, Breite, Tiefe) des Rahmens 21 ist dabei so gewählt, dass die Monitorkomponenten 28 und 30 funktionell zusammenwirkend in den Rahmen 21 einlegbar, elektrisch konnektierbar und an dem Tragelement 20 ortsfest haltbar bzw. festlegbar sind. Das Tragelement 20 selbst ist mittels der Erhebungen 14 an dem Blechgehäuse 10 festlegbar. Der ebenfalls aus Kunststoff bestehende Rahmen 21 trägt als Teil einer dreidimensionalen Formgebung vorteilhaft zur Verwindungssteifigkeit des Tragelements 20 bei, ohne dessen Gesamtgewicht nennenswert zu erhöhen.

Das Dichtungselement 27 ist zwischen der Monitorkomponente 28 und dem Tragelement 20 angeordnet. Es soll die Monitorkomponente 28 vor Beschädigungen durch das Tragelement 20 schützen und das Eindringen von Feuchtigkeit und Schmutz verhindern.

Der hervorstehende Rahmen 21 dient als Versteifung des Tragelements 20 und führt durch eine entsprechende Geometrie das Dichtungselement 27 sowie die Monitorkomponenten 28 und 30. Durch die Monitorkomponente 30, welche über Befestigungselemente 31, die auch als Aufnahme- und Versteifungselemente ausgebildet sein können, mit dem Tragelement 20 verschraubt wird, werden das Dichtungselement 27 sowie die Monitorkomponente 28 geklemmt und dadurch in Ihrer Lage fixiert.

Gemäß dem Ausführungsbeispiel weist die Monitorkomponente 30 eine bestimmte Eigensteifigkeit auf und ist als sozusagen letzte Sandwichkomponente derart auf die zuvor in dem Rahmen 21 aufgenommenen Komponenten 27 und 28, d.h. das Dichtungselement 27 und beispielsweise eine berührungsempfindliche, transparentes und flächige Touchfolie 28, legbar , dass sie diese im aufgelegten Zustand rückseitig flächig hält und abstützt. Am Umfang der Monitorkomponente 30 ist zur Befestigung oder Halterung am Tragelement 20 eine vorbestimmte Anzahl von Befestigungselementen 31 angeordnet. Die Befestigungselemente 31 können beispielsweise laschenfömig und/oder winkelförmig ausgelegt sein, mit einer Durchgangsöffnung, durch welche ein Befestigungselement 31 wie beispielsweise eine Schraube, führbar und in einer vorzugsweise ebenfalls rund- oder stiftförmigen Erhebung 26 an dem Tragelement 20 fixierbar ist. In diesem Ausführungsbeispiel wird an jedem Eckbereich der Monitorkomponente 30 ein Befestigungselement 31 verwendet, mithin insgesamt vier Befestigungselemente 31. Die Anzahl und/oder ein Werkstoff der Befestigungselemente 31 ist/sind jedoch nicht weiter beschränkt, solange ein betriebssicheres und funktionell kompatibles Halten und Abstützen der Monitorkomponenten 28 und 30 gewährleistet ist. So ist beispielsweise auch eine rückseitig der Monitorkomponente 30 zwei Eckbereiche verbindende Diagonalstruktur oder eine vier Eckbereiche oder Mittenbereiche verbindende Kreuzstruktur darstellbar.

Die Monitorkomponente 30 kann mechanische und/oder elektrische Anschlüsse oder Verbindungselemente aufweisen oder ankoppelbar bereitstellen, die eine elektrische Konnektivität für zumindest eine elektronische Baugruppe bzw. ein Trägerelement für elektrische Bauteile 40 mit Bezug zu den Monitorkomponenten 28 und 30 des Monitors bereitstellen. Auf diese Weise kann vorteilhaft gegebenenfalls notwendige Ansteuerelektronik, Verarbeitungselektronik, eine Leistungsversorgung, eine Schnittstelle und/oder dergleichen Teil der gesamten Baugruppe 40 werden, die dann an der Rückseite der Monitorkomponente 30 konnektierbar ist.

Gemäß dem Ausführungsbeispiel ist eine Halterung 32 vorgesehen, die ein Versteifungselement bildet. Die Halterung 32 besteht vorzugsweise aus Metall, das beispielsweise winkelblechförmig ausgestaltet sein kann, und ist dazu angeordnet, das Tragelement 20 mit den in dem Rahmenabschnitt 21 aufgenommenen Komponenten 28 und 30 des Monitors an dem Blechgehäuse 10 festzulegen. Dazu weist die Halterung 32 in diesem Ausführungsbeispiel eine Reihe von Öffnungen 34 auf, die in Größe und Lage zu einem relevanten Teil bzw. einer relevanten Anzahl der Erhebungen 14 und der Öffnungen 24 an dem Tragelement 20 korrespondieren, so dass zunächst das (gegebenenfalls teilweise oder vollständig zu der Monitorbaugruppe vormontierte) Tragelement 20 lose an dem Blechgehäuse 10 platziert werden kann, indem die Erhebungen 14 durch die Öffnungen 24 geführt werden, sodann die Halterung 32 lose an dem Tragelement 20 platziert werden kann, in dem die Erhebungen 14 auch durch die Öffnungen 34 geführt werden, und schließlich eine Verschraubung an der Gewindeanordnung der Erhebungen 14 die Halterung 32 samt darunter liegendem Tragelement 20 an dem bzw. gegen das Blechgehäuse 10 fixiert, beispielsweise mittels einer Schraubverbindung. In anderen Worten ist die Halterung 32 als das zumindest eine Versteifungselement mit zumindest einem Teil der aus Metall bestehenden Erhebungen 14 als den Positionierungs- und/oder Halteelementen des Blechgehäuses 10 verschraubbar. Die Halterung 32 kann in diesem Rahmen ebenfalls dazu ausgelegt sein, gleichzeitig bzw. über dieselben Verschraubungen die elektronische Baugruppe 40 anzulenken und zu halten. Die elektronische Baugruppe 40 kann aber auch am Blechgehäuse 10 montiert und befestigt sein.

Für Anbauteile, wie bspw. die Gehäuserückwand, kann ferner eine alternative Befestigungs-, Abstütz- und/oder Haltevorrichtung vorgesehen sein. Das Tragelement 20 kann dazu rückseitig und von der Grundplatte bzw. Grundfläche aus vorstehend wenigstens eine erste Halteeinrichtung 25 aufweisen, die entlang des Umfangs des Tragelements 20, beispielsweise jeweils in dessen Eckbereichen, angeordnet und so ausgebildet ist, dass daran ein Anbauteil befestigbar ist und/oder das Tragelement 20 an dem Blechgehäuse 10 befestigbar ist.

In einer Ausführungsform der Vorrichtung gemäß dem Ausführungsbeispiel weist die Ausnehmung 22 des Tragelements 20 einen geringeren Umfang auf als eine Ausnehmung 12 des Blechgehäuses 10, so dass stirnseitig des Blechgehäuses 10, d.h. für einen vor dem Gehäuse stehenden Anwender, ein vorbestimmter Abschnitt des Tragelements 20 in der Ausnehmung 12 des Blechgehäuses 10 sichtbar ist.

Es wird angemerkt, dass die Komponenten 28 und 30 des Monitors zumindest ein berührungsempfindliches, transparentes und flächiges Element 28 (Touchfolie) und ein bildgebendes Element 30 (Flüssigkristallanzeige, TFT) beinhalten, die gestapelt in dem Rahmenabschnitt 21 des Tragelements 20 angeordnet sind und einen berührungsempfindlichen Bildschirm 28, 30 ausbilden. Hierzu kann ein insoweit vorgefertigtes Bildschirm- oder Anzeigemodul verwendet werden. Das Tragelement 20, das Dichtungselement 27 sowie die Komponenten 28 und 30 des Monitors werden bevorzugt als system-, geräte- und/oder modellübergreifendes Gleichteil vormontiert und als funktionsfähig anschlussbereite Baugruppe in das Blechgehäuse 10, beispielsweise ein Gehäuse oder Korpus einer Dialysemaschine, gesetzt und mit diesem verbunden.

Wie vorstehend beschrieben wurde, ermöglicht eine Monitorintegration in Sandwich-Bauweise über einen innenliegenden Kunststoffrahmen in ein Blech für Maschinen mit einem Maschinengehäuse aus Blech vorteilhaft eine direkte Monitorintegration in ein bestehendes Blechgehäuse. Durch die erhöhte Funktionsintegration der Bauteile ist eine wirtschaftliche Monitorintegration möglich. Des Weiteren ist eine Gleichteilstrategie durch Standardisierung möglich.

In einer Modifikation des vorstehend beschriebenen Ausführungsbeispiels kann ein reduzierender Subrahmen (Einsatzrahmen, Einsatzstück oder Reduzierstück) (nicht dargestellt) bereitgestellt sein, der die ursprüngliche Größe des Rahmens 21 und/oder der Ausnehmung 22 auf Maße beispielsweise entwicklungsspezifisch vorgesehener oder zu einem bestimmten Zeitpunkt am Markt wirtschaftlich verfügbarer Monitorkomponenten reduzierend anpasst. Ein solcher Subrahmen kann in den (Haupt)Rahmen 21 einlegbar sein und sich an dort angeordneten Stützpunkten oder Stützflächen abstützen, d.h. aufliegen und/oder schnappend bzw. rastend eingreifen, stirnseitig und/oder rückseitig mit einer entsprechend anpassenden Verblendung versehen sein, und insgesamt derart ausgeformt sein, dass die ursprünglich vorhandenen Befestigungseinrichtungen des Tragelements 20 weiter nutzbar bleiben. Der Subrahmen kann dazu vorteilhaft von der Stirn- oder Vorderseite des Tragelements 20 in die Ausnehmung 22 einschiebbar sein, wobei die Blende des Subrahmens in die Fläche des Blechgehäuses 10 erweitert und den in der Ausnehmung 12 des Blechgehäuses gegebenenfalls sichtbaren Rahmenabschnitt des Tragelements 20 mit abdeckt, und wobei eine Einrastvorrichtung des Subrahmens den Rahmen 21 des Tragelements 20 hintergreift und den Subrahmen in dem Rahmen 21 rastend verriegelt. Dadurch können auf gleichbleibender Grundlage, die durch das Tragelement 20, die daran angepasste Monitorkomponente 30 sowie die Halterung 32 gebildet wird, in Abhängigkeit von beispielsweise der Verfügbarkeit von Monitorkomponenten oder Entwicklungsvorgaben, bedarfsweise Monitorkomponenten mit anderer, etwa geringerer, Bildschirmdiagonale oder anderem Bildseitenverhältnis verbaut werden. Dadurch wiederum können innerhalb einer Modellreihe günstigere Grundmodelle mit geringer spezifiziertem Monitor bereitgestellt werden, und/oder sind solche günstigeren Grundmodelle bei steigenden Anforderungen nachträglich erweiterbar, und wird es im Rahmen einer Ersatzteilversorgung möglich, langfristig geeignete Monitorkomponenten bereitzustellen.

Es versteht sich, dass die Erfindung nicht auf das beschriebene Ausführungsbeispiele und dessen Modifikationen beschränkt ist.

## Patentansprüche

1. Baugruppe mit einem Monitor, einem Gehäuse und einer Vorrichtung zur Integration des Monitors in das Gehäuse, wobei die Vorrichtung aufweist:
ein Tragelement (20), das eine Ausnehmung (22) aufweist, die einer vorbestimmten sichtbaren Fläche des Monitors entspricht, wobei das Tragelement (20) aufweist:
eine Vielzahl von Öffnungen (24), die in vorbestimmter Verteilung außerhalb der Ausnehmung (22) zur Durchführung von Positionierungs- und/oder Halteelementen (14) und Platzierung des Tragelements (20) in vorbestimmter Position an dem Gehäuse, in welches das Tragelement (20) aufnehmbar ist, angeordnet sind; und
einen Rahmenabschnitt (21), der gegenüber einer Grundfläche des Tragelements (20) auf einer Rückseite desselben vorsteht und zur Aufnahme von Komponenten (28, 30) des Monitors in Sandwichbauweise angeordnet ist, wobei
die in dem Rahmenabschnitt (21) aufgenommenen Komponenten (28, 30) des Monitors an dem Tragelement (20) festlegbar sind,
das Tragelement (20) an den Positionierungs- und/oder Halteelementen (14) an dem Gehäuse festlegbar ist,
das Tragelement (20) aus einem Kunststoff hergestellt ist und das Gehäuse ein Blechgehäuse (10) ist und
das Tragelement (20) rückseitig vorstehend zumindest eine entlang eines Umfangs des Tragelements (20) angeordnete erste Halteeinrichtung (25) zur schraubbaren Festlegung des Tragelements (20) an dem Blechgehäuse (10) aufweist,
**dadurch gekennzeichnet, dass**
zumindest eine der Komponenten (30) des Monitors mit einer definierten Eigensteifigkeit zum rückseitig flächigen Abstützen der im Rahmenabschnitt (21) aufgenommenen Komponenten (28, 30) des Monitors ausgebildet ist und zumindest einen umfangsseitig angeordneten Festlegeabschnitt (31) aufweist, der dazu angeordnet ist, die zumindest eine zum rückseitig flächigen Abstützen ausgebildete Komponente (30) des Monitors an dem Tragelement (20) festzulegen, und
das Tragelement (20) rückseitig vorstehend zumindest eine entlang eines Umfangs des Rahmenabschnitts (21) angeordnete zweite Halteeinrichtung (26) zur schraubbaren Festlegung der zumindest einen zum rückseitig flächigen Abstützen ausgebildeten Komponente (30) des Monitors aufweist.

2. Baugruppe nach Anspruch 1, bei der die erste Halteeinrichtung (25) ausgebildet ist, zumindest ein Anbauteil und/oder eine Gehäuserückwand an dem Blechgehäuse (10) schraubbar festzulegen.

3. Baugruppe nach Anspruch 1 oder 2, bei der die zumindest eine Komponente (30) des Monitors dazu angeordnet ist, elektrische Konnektivität für zumindest eine elektronische Baugruppe (40) mit Bezug zu den Komponenten (28, 30) des Monitors bereitzustellen.

4. Baugruppe nach einem der vorangehenden Ansprüche, mit zumindest einem Versteifungselement (32), das dazu angeordnet ist, das Tragelement (20) mit den in dem Rahmenabschnitt (21) aufgenommenen Komponenten (28, 30) des Monitors an dem Blechgehäuse (10) festzulegen.

5. Baugruppe nach Anspruch 4, bei der das zumindest eine Versteifungselement (32) dazu angeordnet ist, eine elektronische Baugruppe (40) mit Bezug zu den Komponenten (28, 30) des Monitors festzulegen.

6. Baugruppe nach Anspruch 4 oder 5, bei der das zumindest eine Versteifungselement (32) aus einem Metall besteht und dazu angeordnet ist, schraubbar festgelegt zu werden.

7. Baugruppe nach Anspruch 6, bei der die zumindest eine Komponente (30) des Monitors mit dem Tragelement (20) in Kunststoff verschraubbar ist.

8. Baugruppe nach Anspruch 6, bei der das zumindest eine Versteifungselement (32) mit zumindest einem Teil der Positionierungs- und/oder Halteelemente (14) des Blechgehäuses (10), die aus Metall bestehen, verschraubbar ist.

9. Baugruppe nach einem der vorangehenden Ansprüche, bei der die Ausnehmung (22) des Tragelements (20) einen geringeren Umfang aufweist als eine Ausnehmung (12) des Blechgehäuses (10) derart, dass stirnseitig des Blechgehäuses (10) ein vorbestimmter Abschnitt des Tragelements (20) die Ausnehmung (12) des Blechgehäuses (10) innenseitig rahmend sichtbar ist.

10. Baugruppe nach einem der vorangehenden Ansprüche, bei der die Komponenten (28, 30) des Monitors zumindest ein berührungsempfindliches, transparentes und flächiges Element (28), und ein bildgebendes Element (30) beinhalten, die gestapelt in dem Rahmenabschnitt (21) des Tragelements (20) angeordnet sind und einen berührungsempfindlichen Bildschirm (28, 30) ausbilden.

11. Baugruppe nach einem der vorangehenden Ansprüche, bei der das Tragelement (20), ein Dichtungselement (27), das zwischen Tragelement (20) und Komponente (28) des Monitors angeordnet ist und das dazu angeordnet ist, eine Abdichtwirkung bereitzustellen und/oder Toleranzen auszugleichen, sowie die Komponenten (28, 30) des Monitors dazu angeordnet sind, vormontiert zu sein und als funktionsfähig anschlussbereite Baugruppe in das Blechgehäuse (10) gesetzt und mit diesem verbunden zu werden.

12. Baugruppe nach einem der vorangehenden Ansprüche, mit einem Subrahmen, der in den Rahmen (21) des Tragelements (20) einsetzbar und dazu angeordnet ist, die Größe des Rahmens (21) und/oder der Ausnehmung (22) des Tragelements (20) zu verringern.

13. Dialysemaschine, mit einer Baugruppe nach einem der vorangehenden Ansprüche, wobei das Blechgehäuse (10) ein Gehäuse oder Korpus der Dialysemaschine ist.

## Claims

1. An assembly with a monitor, a case, and a device for integrating the monitor in the case, wherein the device comprises:
a support element (20) having a recess (22) corresponding to a predetermined visible area of the monitor, the support element (20) comprising:
a plurality of apertures (24) arranged at predetermined distribution outside the recess (22) for passing through positioning and/or retaining elements (14) and placing the support element (20) at a predetermined position on the case in which the support element (20) can be received; and
a frame section (21) projecting from a base area of the support element (20) on a rear side thereof and being arranged for receiving components (28, 30) of the monitor in sandwich design, wherein
the components (28, 30) of the monitor received in the frame section (21) can be fixed to the support element (20),
the support element (20) can be fixed to the positioning and/or retaining elements (14) on the case,
the support element (20) is made of a plastic material and the case is a sheet-metal case (10), and
the support element (20) includes, projecting from the rear side, at least a first retaining means (25) located along a periphery of the support element (20) for screw-fixing the support element (20) to the sheet-metal case (10),
**characterized in that** at least one of the components (30) of the monitor is configured to have a defined inherent rigidity for backing the components (28, 30) of the monitor received in the frame section (21) fully on the rear side and includes at least one peripherally arranged fixing section (31) which is arranged to fix the at least one component (30) of the monitor formed for backing from the rear side on the support element (20), and
the support element (20) includes, projecting from the rear side, at least a second retaining means (26) disposed along a periphery of the frame section (21) for screw-fixing the at least one component (30) of the monitor formed for full backing from the rear side.

2. The assembly according to claim 1, wherein the first retaining means (25) is formed for screw-fixing at least one attaching part and/or a rear case wall to the sheet-metal case (10).

3. The assembly according to claim 1 or 2, wherein the at least one component (30) of the monitor is arranged to provide electric connectivity for at least one electronic sub-assembly (40) with respect to the components (28, 30) of the monitor.

4. The assembly according to one of the preceding claims, comprising at least one stiffening element (32) which is arranged to fix the support element (20) with the components (28, 30) of the monitor received in the frame section (21) on the sheet-metal case (10).

5. The assembly according to claim 4, wherein the at least one stiffening element (32) is arranged to fix an electronic sub-assembly (40) with respect to the components (28, 30) of the monitor.

6. The assembly according to claim 4 or 5, wherein the at least one stiffening element (32) is made from metal and is arranged to be fixed by screwing.

7. The assembly according to claim 6, wherein the at least one component (30) of the monitor can be screw-connected to the support element (20) in plastic material.

8. The assembly according to claim 6, wherein the at least one stiffening element (32) can be screw-connected to at least a part of the positioning and/or retaining elements (14) of the sheet-metal case (10) which are made from metal.

9. The assembly according to one of the preceding claims, wherein the recess (22) of the support element (20) has a smaller periphery than a recess (12) of the sheet-metal case (10) such that on the front end of the sheet-metal case (10) a predetermined section of the support element (20) is visible while framing the recess (12) of the sheet-metal case (10) on the inside.

10. The assembly according to one of the preceding claims, wherein the components (28, 30) of the monitor comprise at least one touch-sensitive transparent and flat element (28) and an imaging element (30) which are arranged to be stacked in the frame section (21) of the support element (20) and form a touch-sensitive screen (28, 30).

11. The assembly according to one of the preceding claims, wherein the support element (20), a sealing element (27) sandwiched between the support element (20) and the component (28) of the monitor and arranged to provide a sealing effect and/or to compensate for tolerances as well as the components (28, 30) of the monitor are arranged so as to be pre-assembled and to be inserted as a functionally ready-to-connect sub-assembly in and to be connected to the sheet-metal case (10).

12. The assembly according to one of the preceding claims, comprising a subframe which is adapted to be inserted in the frame (21) of the support element (20) and is arranged to reduce the size of the frame (21) and/or of the recess (22) of the support element (20).

13. A dialysis machine, comprising an assembly according to one of the preceding claims, wherein the sheet-metal case (10) is a case or body of the dialysis machine.

## Revendications

1. Ensemble comprenant un moniteur, un boîtier et un dispositif pour intégrer le moniteur dans le boîtier, dans lequel le dispositif présente :
un élément de support (20) qui présente un évidement (22), qui correspond à une surface visible prédéterminée du moniteur, dans lequel l'élément de support (20) présente :
une pluralité d'ouvertures (24) qui sont disposées selon une répartition prédéterminée à l'extérieur de l'évidement (22) pour le passage d'éléments de positionnement et/ou de maintien (14) et le placement de l'élément de support (20) dans une position prédéterminée sur le boîtier, dans lequel l'élément de support (20) peut être reçu ; et
une section de cadre (21) qui fait saillie d'une surface de base de l'élément de support (20) sur un côté arrière de celui-ci et est disposée pour prendre en sandwich des composants (28, 30) du moniteur, dans lequel
les composants (28, 30) du moniteur reçus dans la section de cadre (21) peuvent être fixés à l'élément de support (20),
l'élément de support (20) peut être fixé aux éléments de positionnement et/ou de maintien (14) sur le boîtier,
l'élément de support (20) est constitué d'une matière plastique et le boîtier est un boîtier en tôle (10) et
l'élément de support (20) présente, en saillie sur le côté arrière, au moins un premier dispositif de maintien (25) disposé le long d'une circonférence de l'élément de support (20) pour fixer par vissage l'élément de support (20) sur le boîtier en tôle (10),
**caractérisé en ce**
**qu'**au moins un des composants (30) du moniteur est conçu avec une rigidité propre définie pour supporter les composants (28, 30) du moniteur reçus dans la section de cadre (21), de manière plane sur le côté arrière, et présente au moins une section de fixation (31) disposée côté circonférentiel, qui est disposée pour fixer l'au moins un composant (30) du moniteur conçu pour le support de manière plane sur le côté arrière à l'élément de support (20), et
l'élément de support (20) en saillie sur le côté arrière présente au moins un second dispositif de maintien (26) disposé le long d'une circonférence de la section de cadre (21) pour fixer par vissage au moins un composant (30) du moniteur conçu pour le support sur le côté arrière.

2. Ensemble selon la revendication 1, dans lequel le premier dispositif de maintien (25) est conçu pour fixer par vissage au boîtier en tôle (10) au moins une pièce rapportée et/ou une paroi arrière de boîtier.

3. Ensemble selon la revendication 1 ou 2, dans lequel l'au moins un composant (30) du moniteur est disposé pour fournir une connectivité électrique pour au moins un ensemble électronique (40) lié aux composants (28, 30) du moniteur.

4. Ensemble selon l'une quelconque des revendications précédentes, avec au moins un élément de raidissement (32) qui est disposé pour fixer l'élément de support (20) avec les composants (28, 30) du moniteur reçus dans la section de cadre (21) au boîtier en tôle (10).

5. Ensemble selon la revendication 4, dans lequel l'au moins un élément de raidissement (32) est disposé pour fixer un ensemble électronique (40) par rapport aux composants (28, 30) du moniteur.

6. Ensemble selon la revendication 4 ou 5, dans lequel l'au moins un élément de raidissement (32) est constitué d'un métal et est disposé pour être fixé par vissage.

7. Ensemble selon la revendication 6, dans lequel l'au moins un composant (30) du moniteur est vissable sur l'élément de support (20) en matière plastique.

8. Ensemble selon la revendication 6, dans lequel l'au moins un élément de raidissement (32) est vissable sur au moins une partie des éléments de positionnement et/ou de maintien (14) du boîtier en tôle (10), qui sont constitués de métal.

9. Ensemble selon l'une quelconque des revendications précédentes, dans lequel l'évidement (22) de l'élément de support (20) présente une circonférence plus petite qu'un évidement (12) du boîtier en tôle (10) de sorte que, sur la face avant du boîtier en tôle (10), une section prédéterminée de l'élément de support (20) est visible en encadrant l'évidement (12) du boîtier en tôle (10) sur le côté intérieur.

10. Ensemble selon l'une quelconque des revendications précédentes, dans lequel les composants (28, 30) du moniteur contiennent au moins un élément tactile transparent et plat (28), et un élément de formation d'image (30), qui sont empilés dans la section de cadre (21) de l'élément de support (20) et forment un écran tactile (28, 30).

11. Ensemble selon l'une quelconque des revendications précédentes, dans lequel l'élément de support (20), un élément d'étanchéité (27) disposé entre l'élément de support (20) et le composant (28) du moniteur et disposé pour fournir une action d'étanchéité et/ou compenser des tolérances, ainsi que les composants (28, 30) du moniteur sont disposés pour être prémontés et placés dans le boîtier en tôle (10) et connectés à celui-ci en tant qu'ensemble fonctionnellement prêt à être connecté.

12. Ensemble selon l'une quelconque des revendications précédentes, avec un sous-cadre qui est insérable dans le cadre (21) de l'élément de support (20) et disposé pour réduire la taille du cadre (21) et/ou de l'évidement (22) de l'élément de support (20).

13. Machine de dialyse, avec un ensemble selon l'une quelconque des revendications précédentes, dans laquelle le boîtier en tôle (10) est un boîtier ou un corps de la machine de dialyse.
